# EUROPEAN PATENT APPLICATION

(11) **EP 4 529 925 A1**
(43) Date of publication of application: **02.04.2025**
(21) Application number: 23199673.7
(22) Date of filing: 26.09.2023
(51) Int. Cl.: A61K 31/573, A61K 9/00, A61P 17/00

(54) **CORTEXOLONE-17-ALPHA- PROPIONATE FOR TREATING ACNEIFORM ERUPTIONS**

(71) Applicant: Cassiopea S.p.A., 20045 Lainate (MI) (IT)
(72) Inventor: LONGO, Luigi, Lainate, Milan (IT); GERLONI, Mara, Lainate, Milan (IT); PAGANI, Stefania, Lainate, Milan (IT)
(74) Representative: De Gregori, Antonella

(57) **Abstract**

The present disclosure provides a method of treating a cutaneous acneiform eruption in a subject in need thereof. The method comprises topically administering to the subject an effective amount of cortexolone-17α-propionate. The acneiform eruption can comprise a papule, a pustule, a nodule, crust, a cyst, or any combination thereof on an affected area of the subject. The effective amount of cortexolone-17α-propionate can be administered in a topical pharmaceutical formulation comprising cortexolone-17α-propionate and one or more pharmaceutically acceptable carriers.

## Description

### BACKGROUND

Acneiform eruptions are a subset of acne rash on the skin characterized by a papulopustular rash on the face, scalp, chest, or upper back that can be specifically induced by certain medications. The most common drugs associated with acneiform eruptions are corticosteroids, neuropsychotherapeutic drugs, anti-tuberculosis drugs, and immunomodulating molecules. Recently, targeted therapeutic drug for oncology such as epidermal growth factor receptor (EGFR) inhibitors and other kinase inhibitors, have been associated with increased frequency of acneiform eruptions. Dermatologic reactions are the most common adverse reactions associated with EGFR inhibitors, affecting up to 90% of patients. Acneiform eruptions can, for example, decrease patient compliance, lead to discontinuation of therapy, affect quality of life, increase risk of infection, and reduce treatment effectiveness.

Acneiform eruptions typically have an acute onset as a mild to moderate skin rash, often resembling acne vulgaris that can escalate to lesions, such as more severe papules, pustules, crusts, and cysts. Therapeutic intervention of acneiform eruption involves appropriate skincare, sun protection, moisturizers, topical corticosteroids, and topical systemic antibiotics. However, acneiform eruptions are particularly resistant to conventional acne therapies. *See* e.g., Brezinski et al., Practical Dermatology: Clinical Focus, 37-40 (August 2013), which is incorporated herein by reference in its entirety.

Thus, there remains a need for safe and effective pharmacological therapies for treating acneiform eruptions, including those induced by targeted therapeutic drugs, such as EGFR inhibitors.

### BRIEF SUMMARY

Cortexolone-17α-propionate, also known as 17α-propionyloxy-21-hydroxy-pregna-4-ene-3,20-dione and clascoterone, is a topical antiandrogen that inhibits the effect of androgenic hormones (e.g., testosterone and its more active metabolite dihydrotestosterone) by preventing the binding of the same at the androgen receptor (AR). Cortexolone-17α-propionate is known to be suitable for treating acne, alopecia, and other diseases of skin and cutaneous appendages. *See,* e.g., U.S. Patent Nos. 8,143,240 and 8,865,690, and PCT publications WO 2009/019138, and WO 2016/207778, each of which is incorporated herein by reference in its entirety. Cortexolone-17α-propionate is also known to exist in several distinct crystalline polymorphs, each having unique properties. *See,* e.g., U.S. Patent No. 8,785,427 the entirety of which is incorporated herein by reference.

It has been surprisingly discovered that acneiform eruptions can be effectively treated with cortexolone-17α-propionate for to treat or prevent rashes and/or palliation. As a result of this therapy, an advantageous clinical result can be realized after daily topical administration for an appropriate amount of time, such as a few days, weeks, or months.

Accordingly, the present disclosure provides a method of treating a cutaneous acneiform eruption in a subject in need thereof comprising topically administering an effective amount of cortexolone-17α-propionate to an affected area of the subject.

In some aspects, the acneiform eruption comprises a papule, a pustule, a nodule, crust, a cyst, or any combination thereof, on the affected area of the subject. In some aspects, the acneiform eruption is a targeted therapeutic drug-induced lesion.

In some aspects, the subject is receiving treatment for cancer. In some aspects, the treatment comprises a targeted therapeutic drug selected from the group consisting of an Epidermal Growth Factor Receptor (EGFR) inhibitor, a Mitogen-Activated Protein Kinase (MEK) inhibitor, a Human Epidermal Growth Factor Receptor (HER-2) inhibitor, a Vascular Endothelial Growth Factor Receptor (VEGFR) inhibitor, a RAS/RAF/MEK/ERK (MAPK) inhibitor, a multikinase inhibitor, a mammalian Target of Rapamycin (mTOR) inhibitor, a Programmed Death (PD-1)/Programmed Death Ligand (PDL-1 and PDL-2) inhibitor, a Cytotoxic T Lymphocyte-Associated Antigen (CTLA-4) inhibitor, an Anaplastic Lymphoma Kinase (ALK) inhibitor, a Raf kinase (RAF) inhibitor, a Phosphoinositide 3-kinase (PI3K) inhibitor, an AKT kinase inhibitor, or any combination thereof. In some aspects, the targeted therapeutic drug is gefitinib, erlotinib, afatinib, osimertinib, dacomitinib, lapatinib, neratinib, avitinib, olmutinib, pelitinib, pyrotinib, brigatinib, vandetanib, icotinib, mobocertinib, almonertinib, lazertinib, alflutinib, rociletinib, nazartinib, naquotinib, cetuximab, panitumumab, necitumumab, amivantamab, trametinib, cobimetinib, pimasertib, mirdametinib, refametinib, binimetinib, selumetinib, trastuzumab, pertuzumab, bevacizumab, aflibercept, vemurafenib, dabrafenib, encorafenib, pictilisib, copanlisib, buparlisib, taselisib, ipatasertib, uprosertib, sorafenib, sunitinib, everolimus, sirolimus, nivolumab, pembrolizumab, ipilimumab, tremelimumab, or any combination thereof.

In some aspects, the cortexolone-17α-propionate is administered in a pharmaceutical formulation comprising a pharmaceutically acceptable carrier. In some aspects, the pharmaceutical formulation is a solution, a suspension, an emulsion, a microemulsion, a cream, a paste, a biogel, a gel, a foam, an ointment, a wash-off agent, a patch, a hydrogel patch, or a pledget. In some aspects, the pharmaceutical formulation comprises at least one additive selected from the group consisting of a solvent, an acid, a buffer, an antioxidant, an emulsifier, a penetration enhancer, a moisturizer, a preservative, a chelating agent, and any combination thereof.

In some aspects, the antioxidant is selected from the group consisting of butylated hydroxytoluene (BHT), butylated hydroxyanisole (BHA), ascorbyl palmitate, ascorbic acid, tocopherol, propyl gallate, 2,4,5-trihydroxybutyrophenone, 4-hydroxymethyl-2,6-di-tert-butylphenol, erythorbic acid, gum guaiac, thiodipropionic acid, dilauryl thiodipropionate, tert-butylhydroquinone, pharmaceutically acceptable salts thereof, esters thereof, and any combination thereof.

In some aspects, the emulsifier is selected from the group consisting of PEG-15 hydroxystearate (polyoxyl-15-hydroxystearate), PEG-30 stearate, PEG-40 laurate, PEG-40 oleate, polysorbate 20, polysorbate 60, polysorbate 80, PEG-20 cetostearyl ether, polyoxyl 25 cetostearyl, glyceryl monostearate, cetomacrogol 1000, sorbitan monolaurate, sorbitan monopalmitate, sorbitan monooleate a propylene glycol ester of a fatty acid, a polyglycerol ester of a fatty acid, polyoxyl 5 castor oil, polyoxyl 15 castor oil, polyoxyl 35 castor oil, polyoxyl 40 hydrogenated castor oil, caprylocapryl polyoxyl-8 glycerides, caprylocaproyl polyoxylglycerides, lauroyl polyoxylglycerides, oleoyl polyoxylglycerides, and any combination thereof. In some aspects, the emulsifier is polysorbate 80, glyceryl monostearate, or a combination of polysorbate 80 and glyceryl monostearate.

In some aspects, the penetration enhancer is selected from the group consisting of a polyol, a fatty acid, a fatty alcohol, a fatty acid ester, a surfactant, a pyrrolidone, and any combination thereof.

In some aspects, the moisturizer is cetyl alcohol, cetearyl alcohol, stearyl alcohol, stearic acid, isopropyl myristate, glyceryl monostearate, isopropyl palmitate, cocoa butter, lanolin, liquid paraffin (e.g., mineral oil), shea butter, a silicone oil, castor oil, a polyethylene glycol, glycerol, lactic acid, glycolic acid, malic acid, citric acid, tartaric acid, or any combination thereof.

In some aspects, the buffer is a phosphate buffer, citrate buffer, lactate buffer, or any combination thereof.

In some aspects, the pharmaceutical formulation comprises cortexolone 17α-propionate, propylene glycol, cetyl alcohol, tocopherol, citric acid (e.g., citric acid monohydrate), edetate disodium, mono- and di-glycerides, liquid paraffin (e.g., mineral oil), polysorbate 80, and water. In certain aspects, the pharmaceutical formulation comprises about 1 wt% cortexolone 17α-propionate, about 25 wt% propylene glycol, about 2.5 wt% cetyl alcohol, about 15 wt% glyceryl monostearate, about 10 wt% liquid paraffin (e.g., mineral oil), about 1 wt% polysorbate 80, and up to about 0.05 wt% tocopherol. In some aspects, the pharmaceutical formulation can also comprise a suitable agent for adjusting the pH, such as citric acid.

In some aspects, the cortexolone-17α-propionate is administered in an amount of about 0.1 to about 5 wt%. In certain aspects, the cortexolone 17α-propionate is administered in an amount of about 1 wt%.

In some aspects, topically applying the cortexolone 17α-propionate comprises administering a formulation comprising cortexolone 17α-propionate dissolved therein and cortexolone 17α-propionate present in at least one of polymorphic Forms I, II, III, and IV, or any combination thereof.

### DETAILED DESCRIPTION

The articles "a," "an," and "the" are used herein to refer to one or to more than one (i.e., to at least one) of the grammatical object of the article. By way of example, "an element" means one element or more than one element.

As used herein, the term "or" is a logical disjunction (*i.e.,* and/or) and does not indicate an exclusive disjunction unless expressly indicated as such with the terms "either," "unless," "alternatively," and words of similar effect.

As used herein, the term "about" means within an acceptable error range for the particular value as determined by one of ordinary skill in the art, which depends in part on how the value is measured or determined, i.e., the limitations of the measurement system. "About" can mean a range of up to 10% (e.g., up to 5%, up to 1%) of a given value.

As used herein, the phrase "acneiform eruption" or "acneiform rash" refers to a skin disease that can be characterized as a papule, a pustule, a nodule, crust, or a cyst.

As used herein, the phrase "affected area" refers to an area of the skin that presents with acneiform eruptions. An affected area can include any skin region on the body, including, e.g., face, arm, armpit, leg, chest, abdomen, back, neck, or any combination of the foregoing.

As used herein, the term "anhydrous" means substantially free of water, i.e., having less than about 5 wt% water, and, in certain aspects as specified herein, less than about 3 wt% water, or less than about 1 wt% water.

As used herein, the term "anti-acneiform agent" refers to an agent which treats, prevents, and/or ameliorates acneiform eruptions including, but not limited to, an antiseptic, an antibiotic, a retinoid, an antioxidant, an organic acid, a corticosteroid, an antiparasitic agent, sulfur, and urea.

As used herein, the term "at least" prior to a number or series of numbers is understood to include the number associated with the term "at least," and all subsequent numbers or integers that could logically be included, as clear from context. When at least is present before a series of numbers or a range, it is understood that "at least" can modify each of the numbers in the series or range. For example, "at least 3" means at least 3, at least 4, at least 5, etc. When at least is present before a component in a method step, then that component is included in the step, whereas additional components are optional.

As used herein, the term "Once Daily" or "OD" means "once a day."

As used herein, the term "Bis In Die" or "BID" means "twice a day."

As used herein, the term "buffer" refers to a compound or group of compounds (e.g., a weak conjugate acid-base pair, such as a weak acid and its conjugate base or a weak base and its conjugate acid) that controls the pH of a solution within a desired range. In some aspects, the conjugate base can be added as a Group I or Group II salt. Examples of a buffer include, e.g., a phosphate buffer, citrate buffer, lactate buffer, or any combination thereof.

As used herein, the phrase "C₁-C₇ alcohol" refers to an alcohol having 1, 2, 3, 4, 5, 6, or 7 carbons that is suitable for use in a topical pharmaceutical formulation. Examples of such C₁-C₇ alcohols can include, but are not limited to, methanol, ethanol, isopropanol, n-butanol, n-propanol, benzyl alcohol, and the like. Without wishing to be bound by any particular theory, it is believed that C₁-C₇ alcohols, and particularly short chain C₁-C₇ alcohols, like C₂-C₃ alcohols (e.g., ethanol, n-propanol, or isopropyl alcohol), contribute to the spreadability of the pharmaceutical formulations described herein.

As used herein, the term "chelating agent" refers to a compound that can bind metal ions or metallic compounds. In some aspects, a chelating agent can stabilize the formulation by preventing, e.g., oxidation or precipitation. Suitable chelating agents include, e.g., citric acid (e.g., citric acid monohydrate, anhydrous citric acid), phytic acid, sodium phytate, clioquinol, ethylenediaminetetraacetic acid (EDTA) or a salt thereof (e.g., disodium EDTA, tetrasodium EDTA), etidronic acid, galactaric acid, tetrahydroxypropyl ethylenediamine, sodium metasilicate, or any combination thereof.

As used herein, the term "co-administration" means concurrent or sequential administration of two different active agents, i.e., cortexolone-17α-propionate and a secondary anti-acneiform agent. In some aspects, the active agents are administered close enough in time to each other that the first and second therapeutic agents are simultaneously present in subjects receiving the co-administration.

As used herein the term "cortexolone" (also known as "11-deoxycortisol" or "Reichstein's substance") refers to the compound having the structure:

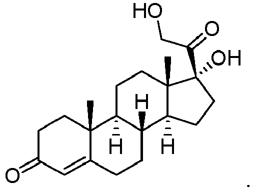

As used herein, the term "cortexolone-17α-propionate" refers to the compound 17α-propionyloxy-21-hydroxy-pregna-4-ene-3,20-dione, which is also known as clascoterone and is equivalent to the chemical structure:

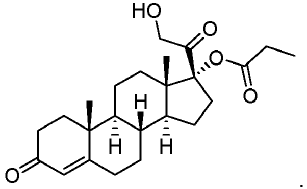

As used herein, the term "degradation product" refers to those compounds that result from the *in vitro* degradation of cortexolone-17α-propionate. Exemplary known cortexolone-17α-propionate degradation products can include, but are not limited to, cortexolone-21-propionate and cortexolone.

The term "effective amount" as used herein refers to an amount of a given active agent that is sufficient, when administered by a method of the disclosure, to efficaciously treat the condition or disease of interest in a subject in need thereof.

As used herein, the term "ethanol" refers to ethyl alcohol, i.e., CH₃CH₂OH, and includes pure (absolute) ethanol and 96% ethanol, the latter being ethanol containing water in an amount typically ranging from about 4% to about 5.1% by volume.

As used herein, the term "fatty" refers to an aliphatic carbon chain that is either saturated or unsaturated. The chain is any suitable length, such as C₄-C₂₈ (e.g., C₄, C₅, C₆, C₇, C₈, C₉, C₁₀, C₁₁, C₁₂, C₁₃, C₁₄, C₁₅, C₁₆, C₁₇, C₁₈, C₁₉, C₂₀, C₂₁, C₂₂, C₂₃, and C₂₄), including more specifically C₁₀-C₂₄, C₁₀-C₂₀, C₁₂-C₂₂, and C₁₂-C₁₈ chain lengths.

As used herein, the term "metabolite" refers to those compounds that result from the *in vivo* metabolism or degradation of an active agent, such as cortexolone-17α-propionate. Exemplary known metabolites of cortexolone-17α-propionate can include, but are not limited to, cortexolone and tetrahydrocortexolone.

As used herein, the term "moisturizer" refers to those compounds that impart moisture to skin, soften skin, or both. Exemplary moisturizers are emollients that include, e.g., a fatty alcohol, a fatty acid, a fatty acid ester, an oil, a polyethylene glycol, glycerol, an alpha hydroxy acid (e.g., lactic acid, glycolic acid, malic acid, citric acid, or tartaric acid), and any combination thereof. Specific examples of a moisturizer include, e.g., cetyl alcohol, cetearyl alcohol, stearyl alcohol, stearic acid, glyceryl monostearate, isopropyl myristate, isopropyl palmitate, cocoa butter, lanolin, liquid paraffin (e.g., mineral oil), shea butter, a silicone oil, castor oil, a polyethylene glycol, glycerol, lactic acid, glycolic acid, malic acid, citric acid, tartaric acid, and any combination thereof.

As used herein the phrase "natural oil" refers to those oils isolable from natural sources. Exemplary natural oils can include, but are not limited to, olive oil, jojoba oil, coconut oil, almond oil, cottonseed oil, safflower oil, sunflower oil, sesame oil, soybean oil, castor oil, calendula oil, and the like.

As used herein, the phrase "penetration enhancer" refers to those pharmaceutically acceptable compounds that increase penetration of the active agent through the dermis. Exemplary penetration enhancers include, e.g., hydroxypropyl β-cyclodextrin, polyoxyethylene alkyl ethers, polyoxyl glycerides, dimethyl sulfoxide, pyrrolidone, N-methyl-2-pyrrolidone, diethylene glycol monoethyl ether (e.g., TRANSCUTOL^{™}, Gattefossé, France), dimethyl isosorbide, diethyl sebacate, azone, menthol, nerol, camphor, methyl salicylate, polysorbate 80 (polyoxyethylene (80) sorbitan monooleate) (e.g., TWEEN^{™} 80, Sigma-Aldrich, St. Louis, MO), sodium dodecyl sulfate (SDS), benzalkonium chloride, polyoxyl 40 hydrogenated castor oil (e.g., CREMOPHOR^{™} RH40, KOLLIPHOR^{™} RH40, BASF, Germany), didecyldimethylammonium bromide (DDAB), didecyltrimethylammonium bromide (DTAB), fatty acids esters (e.g., isopropyl myristate, isopropyl palmitate), fatty acids (e.g., oleic acid, palmitic acid, linoleic acid, and salts thereof), fatty alcohols (e.g., oleyl alcohol, myristyl alcohol, stearyl alcohol), medium-chain triglycerides, and a combination of any of the foregoing.

As used herein, and unless clear from the context in which it is used that it means otherwise, the term "pH" should be considered an "apparent pH" to acknowledge the fact that the behavior of a pH electrode in a complex multicomponent dermatological vehicle which is not totally aqueous in nature cannot be considered a "pH" in the strictest sense.

As used herein, the term "polyol" refers to organic molecules containing two or more hydroxyl groups (e.g., a diol, a triol, or higher). Exemplary polyols can include, but are not limited to, ethylene glycol, diethylene glycol, triethylene glycol, propylene glycol, glycerol, 1,3-butylene glycol, 1,4-butylene glycol, 1-(1-butoxy-2-propoxy)-2-propanol, 2-methyl-2,4-pentanediol, 1,5-pentanediol, 1,6-hexanediol, glycerol, hexanetriol, and the like.

As used herein, the term "polyol ether" refers to a polyol ether suitable for use in a topical pharmaceutical formulation. Exemplary polyol ethers can include, but are not limited to, diethylene glycol monobutyl ether, dipropylene glycol monobutyl ether, polypropylene glycol, polyethylene glycol, polyethylene-polypropylene triblock copolymers, dipropylene glycol, diethylene glycol monoethyl ether, and the like.

As used herein, the term "preservative" refers to a compound with antimicrobial activity, such as an antibacterial or antifungal. Suitable preservatives include, e.g., a paraben (e.g., methyl paraben, ethyl paraben, propyl paraben, butyl paraben), a benzoate (e.g., sodium benzoate), a sorbate (e.g., potassium sorbate), a quaternary ammonium compound (e.g., benzalkonium chloride, benzethonium chloride), an alcohol (e.g., chlorobutanol, benzyl alcohol, ethanol, phenol, m-cresol, chlorocresol), sodium metabisulfite, imidazolidinyl urea, glycerol, glyceryl monostearate, propylene glycol, and any combination thereof.

As used herein, the term "salt" or "pharmaceutically acceptable salt" is intended to include nontoxic salts synthesized from the parent compound which contains a basic or acidic moiety by conventional chemical methods. Lists of suitable salts are found in Remington's Pharmaceutical Sciences, 23rd ed., Elsevier Inc., Academic Press, Cambridge, MA, pages 307-314, 2020, which is incorporated herein by reference in its entirety. For example, the compound can be a salt of an alkali metal (e.g., sodium or potassium), a salt of an alkaline earth metal (e.g., calcium), or a salt of ammonium.

As used herein, the term "solvent" means one or a mixture of more than one pharmaceutically acceptable solvents suitable for topical application, including without limitation, the face, the scalp, the trunk, a shoulder, the back, a forearm, an arm, a limb, and a leg, that is used to solubilize cortexolone-17α-propionate in a formulation described herein.

The phrase "substantially free of" means that a composition contains little no specified ingredient/component, such as less than about 5 wt%, less than about 4 wt%, less than about 3 wt%, less than about 2 wt%, less than about 1 wt%, less than about 0.5 wt%, less than about 0.3 wt%, less than about 0.2 wt%, less than about 0.1 wt%, or about 0 wt% of the specified ingredient.

As used herein "tetrahydrocortexolone" refers to the compound having the Chemical Abstract Service (CAS) Registry Number 68-60-0.

As used herein, the terms "treat," "treating," and "treatment" refer to any indicia of success in the treatment or amelioration of an injury, disease, or condition, including any objective or subjective parameter such as abatement; remission; diminishing of one or more symptoms or making the injury, disease, or condition more tolerable to the subject; slowing in the rate of degeneration or decline; or improving a subject's physical or mental well-being. The treatment or amelioration of symptoms can be based on objective or subject parameters, including the results of a physical examination, neuropsychiatric examination, or psychiatric evaluation.

As used herein, the term "preventing" refers to keeping from happening or existing, or alternatively delaying the onset or recurrence of a disease, disorder, or condition to which such term applies, or of one or more symptoms associated with a disease, disorder, or condition. The term "preventing" also refers to reducing the incidence of a disease, disorder, or condition. The term "prevention" refers to the act of preventing.

As used herein, the term "sequentially" means that a first active agent is co-administered to a subject in a first period of time followed by an administration of a second active agent in a second period of time. In other words the term "sequentially" includes a first therapy in a first period of time followed by a second therapy in a second period of time. The first and second time periods can be separated on the order of seconds, minutes, hours, or days, as needed. In some aspects, the first and second time periods are separated by seconds (e.g., about 60 seconds or less, about 30 seconds or less).

As used herein, the term "subject" typically is directed to a mammal. In some aspects, the subject to be treated is a human.

As used herein, the phrase "wt%" is intended to encompass and disclose aspects wherein the wt% is percentage of weight by total weight (w/w) for a given value, unless otherwise specified.

As used herein, the terms "comprises," "comprising," "having," "including," "containing," and the like are open-ended terms meaning "including, but not limited to." To the extent a given aspect disclosed herein "comprises" certain elements, it should be understood that present disclosure also specifically contemplates and discloses aspects that "consist essentially of" those elements and that "consist of" those elements.

As used herein the terms "consists essentially of," "consisting essentially of," and the like are to be construed as a semi-closed terms, meaning that no other ingredients which materially affect the basic and novel characteristics of an aspect are included.

As used herein, the terms "consists of," "consisting of," and the like are to be construed as closed terms, such that an aspect "consisting of" a particular set of elements excludes any element, step, or ingredient not specified in the aspect.

### The Method

The present disclosure provides a method of treating a cutaneous acneiform eruption (also known as acneiform rash) in a subject in need thereof comprising topically administering an effective amount of cortexolone-17α-propionate to an affected area of the subject.

In some aspects, the subject can be a mammal, such as a human.

In some aspects, cortexolone-17α-propionate can be applied to any suitable body surface in need of treatment, such as a skin region susceptible to acneiform eruptions. Suitable skin regions include e.g., face, arm, armpit, forearm, leg, shoulder, trunk, chest, abdomen, back, neck, or any combination of the foregoing. In some aspects, the formulation is not delivered to the face. In other aspects, the formulation can be applied to the face.

Acneiform eruptions comprise papules, pustules, nodules, crusts, or cysts, or any combination thereof. Lesions can be painful and itchy but differ from acne vulgaris in that acneiform eruptions typically do not have comedones.

Table 1 depicts the National Cancer Institute - US Common Terminology Criteria for Adverse Events (NCI-CTCAE), which provides a grading of the severity of acneiform eruptions.

**TABLE 1**

| Grade | |
|---|---|
| 1 | Papules and/or pustules covering <10% of the BSA* associated or not associated with symptoms of pruritus or tenderness |
| 2 | Papules and/or pustules covering 10-30% of the BSA associated or not associated with symptoms of pruritus or tenderness; psychosocial impact; limiting instrumental ADL |
| 3 | Papules and/or pustules covering >30% of the BSA associated or not associated with symptoms of pruritus or tenderness; limiting self-care ADL, associated with local superinfection (oral antibiotics indicated) |
| 4 | Covering any percentage of the BSA associated or not with pruritus or tenderness; associated with severe superinfection (intravenous antibiotics indicated); life-threatening consequences |
| 5 | Death |

| | |
|---|---|
| *BSA = body surface area; ADL = activity of daily living | |

In some aspects, the acneiform eruption can be scored a Grade 1, 2, 3, 4, or 5. In some aspects, the acneiform eruption can be scored a Grade 1. In some aspects, the acneiform eruption can be scored a Grade 2. In some aspects, the acneiform eruption can be scored a Grade 3. In some aspects, the acneiform eruption can be scored a Grade 4. In some aspects, the acneiform eruption can be scored a Grade 5.

Acneiform eruption (e.g., acneiform rash) can be caused by several conditions, including drug reactions, infections (e.g., bacterial infection, fungal infection), hormonal or metabolic abnormalities, genetic disorders (e.g., tuberous sclerosis, eruptive vellus hair cysts), contact with chemicals, or mechanical actions. In some aspects, the acneiform can be induced by occupational exposure, chemical exposure, mechanical exposure, or targeted therapeutic drug-exposure.

In some aspects, the acneiform eruptions can be an occupational exposure-induced lesions. For example, occupational exposure can include exposure to a chemical, such as a halogenated chemical (e.g., chloraphthalene, chlorophenyl, chlorophenol).

In some aspects, the acneiform eruptions can be chemical exposure-induced lesions. For example, chemical exposure can include exposure to an oil (e.g., a heavy oil, vegetable oil), wax, coal tar or a derivative there (e.g., pitch, creosote). Exposure can be from a cosmetic or other topical product containing such chemical.

In some aspects, the acneiform eruptions can be mechanical exposure-induced lesions. For example, mechanical exposure can include friction, pressure, or both, particularly from repeated contact. Exposure can be from, e.g., restrictive clothing, prolonged immobilization, and repetitive mechanical trauma.

In some aspects, the acneiform eruption can be a targeted therapeutic drug-induced lesion. The targeted therapeutic drug can be, for example, an antibiotic (e.g., a penicillin, a tetracycline, a macrolide, co-trimoxazole, doxycycline, ofloxacin, chloramphenicol), a corticosteroid, an anticonvulsant (e.g., phenytoin), an antidepressant (e.g., amineptine), an antipsychotic (e.g., olanzapine, lithium), an antituberculosis agent (e.g., isoniazid (INH), thiourea, thiouracil, disulfiram, corticotropin), an antifungal (e.g., nystatin, itraconazole), an anticancer agent, hydroxychloroquine, naproxen, or mercury.

In some aspects, the subject can be receiving treatment for cancer, e.g., treatment with one or more anticancer agents. In some aspects, the anticancer agent can comprise a targeted therapeutic drug selected from the group consisting of an Epidermal Growth Factor Receptor (EGFR) inhibitor, a Mitogen-Activated Protein Kinase (MEK) inhibitor, a Human Epidermal Growth Factor Receptor (HER-2) inhibitor, a Vascular Endothelial Growth Factor Receptor (VEGFR) inhibitor, a RAS/RAF/MEK/ERK (MAPK) inhibitor, a multikinase inhibitor, a mammalian Target of Rapamycin (mTOR) inhibitor, a Programmed Death (PD-1)/Programmed Death Ligand (PDL-1 and PDL-2) inhibitor, a Cytotoxic T Lymphocyte-Associated Antigen (CTLA-4) inhibitor, an Anaplastic Lymphoma Kinase (ALK) inhibitor, a Raf kinase (RAF) inhibitor, a Phosphoinositide 3-kinase (PI3K) inhibitor, and an AKT kinase inhibitor, or any combination thereof.

Suitable EGFR inhibitors can include, but are not limited to, afatinib, alflutinib, almonertinib, brigatinib, cetuximab, dacomitinib, erlotinib, gefitinib, icotinib, lapatinib, lazerinib, mobocertinib, necitumumab, neratinib, olmutinib, osimertinib, panitumumab, pyrotinib, simotinib, and vandetanib.

Suitable MEK inhibitors can include, but are not limited to, binimetinib, cobimetinib, mirdametinib, pimasertib, refametinib, selumetinib, and trametinib.

Suitable HER-2 inhibitors can include, but are not limited to, dacomitinib, lapatinib, margetuximab, neratinib, pertuzumab, trastuzumab, and tucatinib.

Suitable VEGFR inhibitors can include, but are not limited to, axitinib, bevacizumab, cabozantinib, lenvatinib, pazopanib, ponatinib, ramucirumab, regorafenib, sorafenib, sunitinib, tivozanib, and vandetanib.

Suitable MAPK inhibitors can include, but are not limited to, binimetinib, cobimetinib, and trametinib.

Suitable multikinase inhibitors can include, but are not limited to, afatinib, alectinib, avapritinib, axitinib, binimetinib, brigatinib, cabozantinib, capmatinib, ceritinib, cobimetinib, crizotinib, dabrafenib, dasatinib, deucravacitinib, encorafenib, entrectinib, erdafitinib, fedratinib, futibatinib, gilteritinb, imatinib, infigratinib, larotrectinib, lenvatinib, lorlatinib, midostaurin, nintedanib, pacritinib, pazopanib, pemigatinib, pexidartinib, ponatinib, pralsetinib, regorafenib, ripretinib, ritlecitinib, ruxolitinib, selpercatinib, selumetinib, sorafenib, sunitinib, tepotinib, trametinib, umbralisib, vandetanib, and vemurafenib.

Suitable mTOR inhibitors can include, but are not limited to, everolimus, temsirolimus, and sirolimus.

Suitable PD-1/PDL-1 inhibitors can include, but are not limited to, atezolizumab, avelumab, cemiplimab, durvalumab, nivolumab, and pembrolizumab.

Suitable CTLA-4 inhibitors include, but are not limited to ipilimumab and tremelimumab.

Suitable ALK inhibitors can include, but are not limited to, alectinib, alkotinib, belizatinib, brigatinib, certinib, conteltinib, crizotinib, ensartinib, entrectinib, fortinib, lorlatinib, reprotrectinib, and zotizalkib,

Suitable RAF inhibitors can include, but are not limited to, agerafenib, avutometinib, dabrafenib, encorafenib, lifirafenib, sorafenib, tovorafenib, and vemurafenib.

Suitable PI3K inhibitors can include, but are not limited to alpelisib, apitolisib, bimiralisib, buparlisib, copanlisib, dactolisib, duvelisib, eganelisib, fimepinostat, gedatolisib, idelalisib, linperlisib, leniolisib, omipalisib, parsaclisib, paxalisib, pictilisib, pilaralisib, rigosertib, samotolisib, serabelisib, sonolisib, taselisib, tenalisisb, umbralisib, voxtalisib, wortmanin, and zandelisisb.

Suitable AKT kinase inhibitors can include, but are not limited to, afuresertib, capivasertib, ipatasertib, miransertib, perifosine, and uprosertib.

In some aspects, the targeted therapeutic drug can be gefitinib, erlotinib, afatinib, osimertinib, dacomitinib, lapatinib, neratinib, avitinib, olmutinib, pelitinib, pyrotinib, brigatinib, vandetanib, icotinib, mobocertinib, almonertinib, lazertinib, alflutinib, rociletinib, nazartinib, naquotinib, cetuximab, panitumumab, necitumumab, amivantamab, trametinib, cobimetinib, pimasertib, mirdametinib, refametinib, binimetinib, selumetinib, trastuzumab, pertuzumab, bevacizumab, aflibercept, vemurafenib, dabrafenib, encorafenib, pictilisib, copanlisib, buparlisib, taselisib, ipatasertib, uprosertib, sorafenib, sunitinib, everolimus, sirolimus, nivolumab, pembrolizumab, ipilimumab, tremelimumab, or any combination thereof. In some aspects, the targeted therapeutic drug can be afatinib, alflutinib, almonertinib, brigatinib, cetuximab, dacomitinib, erlotinib, gefitinib, icotinib, lapatinib, lazerinib, mobocertinib, necitumumab, neratinib, olmutinib, osimertinib, panitumumab, pyrotinib, simotinib, vandetanib, binimetinib, cobimetinib, mirdametinib, pimasertib, refametinib, selumetinib, trametinib, or any combination thereof.

### Pharmaceutical Formulations

The present disclosure provides a method of treating a cutaneous acneiform eruption (e.g., acneiform rash) in a subject in need thereof comprising topically administering an effective amount of cortexolone-17α-propionate to an affected area of the subject. Topical administration comprises application of cortexolone-17α-propionate to the skin of the subject, as described herein.

In any of the aspects described herein, the cortexolone 17α-propionate to be administered can be at least one of polymorphic Forms I, II, III, and IV. In some aspects, the cortexolone 17α-propionate can be polymorphic Form I. In some aspects, the cortexolone 17α-propionate can be polymorphic Form II. In some aspects, the cortexolone 17α-propionate can be polymorphic Form III. In some aspects, the cortexolone 17α-propionate can be polymorphic Form IV. In some aspects, the cortexolone 17α-propionate can be a mixture of polymorphic Forms I and II. In some aspects, the cortexolone 17α-propionate can be a mixture of polymorphic Forms I and III. In some aspects, the cortexolone 17α-propionate can be a mixture of polymorphic Forms I and IV. In some aspects, the cortexolone 17α-propionate can be a mixture of polymorphic Forms II and III. In some aspects, the cortexolone 17α-propionate can be a mixture of polymorphic Forms II and IV. In some aspects, the cortexolone 17α-propionate can be a mixture of polymorphic Forms III and IV. In some aspects, the cortexolone 17α-propionate can be a mixture of polymorphic Forms I, II, and III. In some aspects, the cortexolone 17α-propionate can be a mixture of polymorphic Forms I, III, and IV. In some aspects, the cortexolone 17α-propionate can be a mixture of polymorphic Forms II, III, and IV. In some aspects, the cortexolone 17α-propionate can be a mixture of polymorphic Forms I, II, III, and IV.

In some aspects, the cortexolone-17α-propionate can be administered in a pharmaceutical formulation comprising a pharmaceutically acceptable carrier. The pharmaceutical formulation can be in any form suitable for topical administration. In some aspects, the pharmaceutical formulation can be a liquid or semi-solid formulation depending on the carriers and excipients that can be present, as described herein. Examples of the liquid or semi-solid formulation include, e.g., a solution, a suspension, an emulsion, a microemulsion, a cream, a paste, a biogel, a gel, a foam, an ointment, a wash-off agent, a patch, a hydrogel patch, or a pledget.

In some aspects, the formulation can be a cream. In some aspects, the cream can comprise equal parts oil and water in the form of an emulsion (i.e., an emulsified cream).

In some aspects, the pharmaceutical formulation can comprise a solvent selected from the group comprising water, a C₁-C₇ alcohol, a polyol ether, a polyol, polyvinyl alcohol (PVA), polyvinyl pyrrolidone (PVP), a natural oil, an ester, tricaprylin (2,3-di(octanoyloxy)propyl octanoate), a medium-chain triglyceride, caprylocaproyl polyoxyl-8 glycerides, or any combination thereof. In some aspects, the one or more pharmaceutically acceptable carriers are selected from the group consisting of water, a polyol, and a combination thereof.

In some aspects, the polyol ether can be selected from the group comprising or the group consisting of polyethylene glycol, polypropylene glycol, polyethylene-polypropylene triblock copolymers, dipropylene glycol, diethylene glycol monoethyl ether (TRANSCUTOL^{™}, Gattefossé, France), and any combination thereof.

When the polyol ether can be a polyethylene glycol (PEG), the PEG can have a number average molecular weight (Mₙ) that can be about 200 g/mol to about 6,000 g/mol. In some aspects, the PEG can be selected from the group comprising or the group consisting of: PEG 200, PEG 300, PEG 400, PEG 540, and PEG 600. In some aspects, the polyol ether can be PEG 200. In other aspects, the polyol ether can be PEG 400. In still other aspects, the polyol ether can be diethylene glycol monoethyl ether.

In some aspects, the polyol (e.g., a diol, a triol, or higher) can be selected from the group comprising or the group consisting of diethylene glycol, triethylene glycol, propylene glycol, ethylene glycol, glycerol, 1,3-butylene glycol, 1,4-butylene glycol, 1-(1-butoxy-2-propoxy)-2-propanol, 2-methyl-2,4-pentanediol, 1,5-pentanediol, 1,6-hexanediol, glycerol, hexanetriol, and any combination thereof. In some aspects, the polyol can be glycerol. In other aspects, the polyol can be propylene glycol.

In some aspects, the polyol can be present in an amount ranging from about 5 wt% to about 55 wt% of the total formulation; from about 10 wt% to about 50 wt% of the total formulation; from about 20 wt% to about 45 wt% of the total formulation; from about 25 wt% to about 40 wt%; or from about 20 wt% to about 30 wt%. In a particular aspect, the polyol can comprise about 30 wt% of the total formulation. In a further aspect, the polyol comprising about 25 wt% of the total formulation can be propylene glycol.

In some aspects, the pharmaceutical formulation can comprise at least about 50 wt% solvent (e.g., water, saline, a buffered saline solution, a polyol, a polyol ether, a C₁-C₇ alcohol, or any combination thereof). In other aspects, the pharmaceutical formulation can comprise at least about 60 wt% solvent. In other aspects, the pharmaceutical formulation can comprise at least about 70 wt% solvent. In other aspects, the pharmaceutical formulation can comprise at least about 80 wt% solvent. In other aspects, the pharmaceutical formulation can comprise at least about 85 wt% solvent, at least about 90 wt% solvent, at least about 91 wt% solvent, at least about 92 wt% solvent; at least about 93 wt% solvent; at least about 94 wt% solvent; or at least about 95 wt% solvent.

In some aspects, the pharmaceutical formulation can further comprise at least one additive selected from an acid, a buffer, an antioxidant, an emulsifier, a penetration enhancer, a moisturizer, a preservative, a chelating agent, and any combination thereof.

In some aspects, the pharmaceutical formulation can comprise from about 0.01 wt% to about 50 wt% of each additive (e.g., an acid, a buffer, an antioxidant, an emulsifier, a penetration enhancer, a moisturizer, a preservative, a chelating agent) relative to the total weight of the composition (i.e., making up 100 wt%). For example, each additive can be present in the pharmaceutical formulation in an amount from about 0.01 wt% to about 45 wt%, about 0.01 wt% to about 40 wt%, about 0.01 wt% to about 35 wt%, about 0.01 wt% to about 30 wt%, about 0.01 wt% to about 25 wt%, about 0.01 wt% to about 20 wt%, about 0.01 wt% to about 15 wt%, about 0.01 wt% to about 10 wt%, about 0.01 wt% to about 5 wt%, about 0.01 wt% to about 2 wt%, about 0.05 wt% to about 50 wt%, about 0.05 wt% to about 45 wt%, about 0.05 wt% to about 40 wt%, about 0.05 wt% to about 35 wt%, about 0.05 wt% to about 30 wt%, about 0.05 wt% to about 25 wt%, about 0.05 wt% to about 20 wt%, about 0.05 wt% to about 15 wt%, about 0.05 wt% to about 10 wt%, about 0.05 wt% to about 5 wt%, about 0.05 wt% to about 2 wt%, about 0.1 wt% to about 50 wt%, about 0.1 wt% to about 45 wt%, about 0.1 wt% to about 40 wt%, about 0.1 wt% to about 35 wt%, about 0.1 wt% to about 30 wt%, about 0.1 wt% to about 25 wt%, about 0.1 wt% to about 20 wt%, about 0.1 wt% to about 15 wt%, about 0.1 wt% to about 10 wt%, about 0.1 wt% to about 5 wt%, about 0.1 wt% to about 2 wt%, about 0.5 wt% to about 50 wt%, about 0.5 wt% to about 45 wt%, about 0.5 wt% to about 40 wt%, about 0.5 wt% to about 35 wt%, about 0.5 wt% to about 30 wt%, about 0.5 wt% to about 25 wt%, about 0.5 wt% to about 20 wt%, about 0.5 wt% to about 15 wt%, about 0.5 wt% to about 10 wt%, about 0.5 wt% to about 5 wt%, about 0.5 wt% to about 2 wt%, about 1 wt% to about 50 wt%, about 1 wt% to about 45 wt%, about 1 wt% to about 40 wt%, about 1 wt% to about 35 wt%, about 1 wt% to about 30 wt%, about 1 wt% to about 25 wt%, about 1 wt% to about 20 wt%, about 1 wt% to about 15 wt%, about 1 wt% to about 10 wt%, about 1 wt% to about 5 wt%, about 1 wt% to about 2 wt%, about 50 wt%, about 48 wt%, about 46 wt%, about 44 wt%, about 42 wt%, about 40 wt%, about 38 wt %, about 36 wt%, about 34 wt%, about 2 wt%, about 30 wt%, about 28 wt%, about 26 wt%, about 24 wt%, about 22 wt%, about 20 wt%, about 18 wt%, about 16 wt%, about 14 wt%, about 12 wt%, about 10 wt%, about 8 wt%, about 6 wt%, about 5 wt%, about 4 wt%, about 3 wt%, about 2 wt%, about 1 wt%, about 0.9 wt%, about 0.8 wt%, about 0.7 wt%, about 0.6 wt%, about 0.5 wt%, about 0.4 wt%, about 0.3 wt%, about 0.2 wt%, about 0.1 wt%, about 0.09 wt%, about 0.08 wt%, about 0.07 wt%, about 0.06 wt%, about 0.05 wt%, about 0.04 wt%, about 0.03 wt%, about 0.02 wt%, or about 0.01 wt% of the total weight of the composition.

In some aspects, the pharmaceutical formulation can include an acid to help solubilize one or both of the active agents, to maintain a desired pH, or both. Any suitable acid can be used, such as a mineral acid (e.g., hydrochloric acid, sulfuric acid, nitric acid, or phosphoric acid), an organic acid (e.g., citric acid, acetic acid, succinic acid, or maleic acid), or mixtures thereof. Examples of suitable acid additives include, e.g., 1-hydroxy-2-naphthoic acid; 2,2-dichloroacetic acid; 2-hydroxyethanesulfonic acid; 2-oxoglutaric acid; 4-acetamidobenzoic acid; 4-aminosalicylic acid; acetic acid; adipic acid; L-ascorbic acid; L-aspartic acid; benzenesulfonic acid; benzoic acid; (+)-camphoric acid; (+)-camphor-10-sulfonic acid; capric acid (decanoic acid); caproic acid (hexanoic acid); caprylic acid (octanoic acid); carbonic acid; cinnamic acid; citric acid (e.g., citric acid monohydrate, anhydrous citric acid); cyclamic acid; dodecylsulfuric acid; ethane-1,2-disulfonic acid; ethanesulfonic acid; formic acid; fumaric acid; galactaric acid; gentisic acid; D-glucoheptonic acid; D-gluconic acid; D-glucuronic acid; glutamic acid; glutaric acid; glycerophosphoric acid; glycolic acid; hippuric acid; hydrobromic acid; hydrochloric acid; isobutyric acid; lactic acid; lactobionic acid; lauric acid; maleic acid; L-malic acid; malonic acid; mandelic acid; methanesulfonic acid; naphthalene-1,5-disulfonic acid; naphthalene-2-sulfonic acid; nicotinic acid; nitric acid; oleic acid; oxalic acid; palmitic acid; pamoic acid; phosphoric acid; proprionic acid; L-pyroglutamic acid; salicylic acid; sebacic acid; stearic acid; succinic acid; sulfuric acid; L-tartaric acid; thiocyanic acid; p-toluenesulfonic acid; and undecylenic acid. In some aspects, acetic acid, citric acid (e.g., citric acid monohydrate, anhydrous citric acid), or lactic acid can be added to the pharmaceutical formulation.

In some aspects, the acid can be added in a sufficient amount so that the pH of the pharmaceutical formulation can be about 7.0 or less (e.g., about 4 to about 7.0, about 4 to about 6.5, about 4 to about 6, about 4 to about 5.5, about 4 to about 5, about 4.5 to about 7.0, about 4.5 to about 6.5, about 4.5 to about 6, about 4.5 to about 5.5, about 4.5 to about 5, about 5 to about 7.0, about 5 to about 6.5, about 5 to about 6, about 5 to about 5.5, about 5.5 to about 7.0, about 5.5 to about 6.5, about 5.5 to about 6, about 6 to about 7.0, or about 6 to about 6.5).

In some aspects, the pharmaceutical formulation can include a buffer to help maintain the pH of the formulation within a desired range. In some aspects, the buffer can be a phosphate buffer, citrate buffer, lactate buffer, or any combination thereof. In some aspects, the formulation can comprise phosphoric acid, citric acid, lactic acid, or any combination thereof.

Because oxidation can result in degradation of an active agent, in some aspects, the pharmaceutical formulation can include an antioxidant. In some aspects, the antioxidant can be, e.g., butylated hydroxytoluene (BHT), butylated hydroxyanisole (BHA), ascorbyl palmitate, ascorbic acid, tocopherol, vitamin E, propyl gallate, 2,4,5-trihydroxybutyrophenone, 4-hydroxymethyl-2,6-di-tertbutylphenol, erythorbic acid, gum guaiac, thiodipropionic acid, dilauryl thiodipropionate, tert-butylhydroquinone, pharmaceutically acceptable salts or esters thereof, and any combination thereof. In some aspects, the antioxidant can be ascorbyl palmitate, BHA, BHT, or any combination thereof. In some aspects, the antioxidant can be BHT, ascorbyl palmitate, or a combination of BHT and ascorbyl palmitate. In some aspects, the antioxidant can be tocopherol (e.g., vitamin E).

In some aspects, the pharmaceutical formulation can include up to about 5 wt% (e.g., up to about 4 wt%, up to about 3 wt%, up to about 2 wt%, or up to about 1 wt%) of an antioxidant. In some aspects, the pharmaceutical formulation can include up to about 0.05 wt% of an antioxidant or include about 0.05 wt% antioxidant.

In some aspects, the pharmaceutical formulation can include an emulsifier, particularly to provide a solution, a suspension, an emulsion, or a microemulsion. Without wishing to be bound to any particular theory, it is believed that emulsifiers, when present, assist or facilitate dissolution of any solid substances, such as an active agent, in the pharmaceutical formulation. In other aspects, however, the emulsifier can be present to facilitate incorporation of two non-miscible liquids into each other (e.g., to form an emulsion or microemulsion).

In other aspects, and without wishing to be bound by any particular theory, an emulsifier can increase product spreadability. For example, and without wishing to be bound to a particular theory, when the pharmaceutical formulation is a liquid solution, the presence of a suitable amount of an emulsifier is believed to decrease the surface tension between the pharmaceutical formulation and the lipid environment of the superficial layer of the skin. This makes it easier to spread the pharmaceutical formulation and is believed to assist penetration of the active agent(s) into skin.

In some aspects, the emulsifier can be selected from the group comprising or the group consisting of: polyethylene glycol (PEG)-fatty acid monoesters such as PEG-15 hydroxystearate (also known as polyoxyl-15-hydroxystearate), PEG-30 stearate, PEG-40 laurate, PEG-40 oleate and the like; polyoxyethylene sorbitan fatty acid esters (polysorbates) such as polysorbate 20 (polyoxyethylene (20) sorbitan monooleate), polysorbate 60 (polyoxyethylene (60) sorbitan monooleate), polysorbate 80 (polyoxyethylene (80) sorbitan monooleate), and the like; polyoxyethylene alkyl ethers such as PEG-20 cetostearyl ether, polyoxyl 25 cetostearyl, glyceryl monostearate, cetomacrogol 1000 and the like; sorbitan fatty acid esters such as sorbitan monolaurate, sorbitan monopalmitate, sorbitan monooleate, and the like; a propylene glycol ester of a fatty acid; a polyglycerol ester of a fatty acid; polyoxyethylene castor oil derivatives such as polyoxyl 5 castor oil, polyoxyl 15 castor oil, polyoxyl 35 castor oil, polyoxyl 40 hydrogenated castor oil and the like; caprylocapryl polyoxyl-8 glycerides; polyoxylglycerides such as caprylocaproyl polyoxylglycerides, lauroyl polyoxylglycerides, oleoyl polyoxylglycerides, and the like; along with a combination of any of the foreoging. In some aspects, the emulsifier can be polyoxyl 40 hydrogenated castor oil, polyoxyl-15-hydroxystearate, polysorbate 60, polysorbate 80, or any combination thereof. In some aspects, the emulsifier can be polysorbate 80, glyceryl monostearate, or a combination of polysorbate 80 and glyceryl monostearate.

In some aspects, the pharmaceutical formulation can include the emulsifier in an amount of up to about 20 wt% of the total formulation (e.g., up to about 19 wt%, up to about 18 wt%, up to about 17 wt%, up to about 16 wt%, up to about 15 wt%, up to about 14 wt%, up to about 13 wt%, up to about 12 wt%, up to about 11 wt%, up to about 10 wt%, up to about 9 wt%, up to about 8 wt%, up to about 7 wt%, up to about 6 wt%, up to about 5 wt%, up to about 4 wt%, up to about 3 wt%, up to about 2 wt%). In some aspects, the emulsifier can be present in an amount ranging from 1 wt% to 20 wt% (e.g., from about 1 wt% to about 19 wt%, from about 1 wt% to about 18 wt%, from about 1 wt% to about 17 wt%, from about 1 wt% to about 16 wt%, from about 1 wt% to about 15 wt%, from about 1 wt% to about 14 wt%, from about 1 wt% to about 13 wt%, from about 1 wt% to about 12 wt%, from about 1 wt% to about 11 wt%, from about 1 wt% to about 10 wt%, from about 1 wt% to about 9 wt%, from about 1 wt% to about 8 wt%, from about 1 wt% to about 7 wt%, from about 1 wt% to about 6 wt%, from about 1 wt% to about 5 wt%, from about 1 wt% to about 4 wt%, from about 1 wt% to about 3 wt%, from about 1 wt% to about 2 wt%, from about 5 wt% to about 20 wt%, from about 5 wt% to about 19 wt%, from about 5 wt% to about 18 wt%, from about 5 wt% to about 17 wt%, from about 5 wt% to about 16 wt%, from about 5 wt% to about 15 wt%, from about 10 wt% to about 20 wt%, from about 10 wt% to about 19 wt%, from about 10 wt% to about 18 wt%, from about 10 wt% to about 17 wt%, from about 10 wt% to about 16 wt%, or from about 10 wt% to about 15 wt%,). In some aspects, the pharmaceutical formulation can include the emulsifier in an amount of about 1 wt%, about 2 wt%, about 3 wt%, about 4 wt%, about 5 wt%, about 6 wt%, about 7 wt%, about 8 wt%, about 9 wt%, about 10 wt%, about 11 wt%, about 12 wt%, about 13 wt%, about 14 wt%, about 15 wt%, about 16 wt%, about 17 wt%, about 18 wt%, about 19 wt%, or about 20 wt%. In still further aspects, the pharmaceutical formulation can include the emulsifier in an amount up to about 20 wt% or it can include about 15 wt% emulsifier.

In some aspects, the pharmaceutical formulation can include one or more penetration enhancers. Without wishing to be bound by a particular theory, penetration enhancers are believed to improve the absorption of the active agent(s) into the skin. Advantageously, and in some aspects, the solvent or some component(s) of the solvent act as a penetration enhancer. For example, in aspects wherein the pharmaceutical formulation comprises ethanol and/or diethylene glycol monoethyl ether, these solvents can also act to enhance penetration of the active agent into the skin. The presence of ethanol and/or diethylene glycol monoethyl ether notwithstanding, the pharmaceutical formulations described herein can further include additional penetration enhancers.

In some aspects, the penetration enhancer can be selected from the group consisting of hydroxypropyl β- cyclodextrin, a polyol, a fatty acid (e.g., myristic acid, palmitic acid, palmitoleic acid, stearic acid, oleic acid, linoleic acid, linolenic acid, or any combination thereof), a fatty alcohol, a fatty acid ester, a surfactant, a pyrrolidone, and any combination thereof. Examples of further penetration enhancer that can be incorporated into formulations described herein can include, but are not limited to, polyoxyethylene alkyl ethers, polyoxyl glycerides, dimethyl sulfoxide, pyrrolidone, N-methyl-2-pyrrolidone, diethylene glycol monoethyl ether (e.g., TRANSCUTOL^{™}, Gattefossé, France), dimethyl isosorbide, diethyl sebacate, azone, menthol, nerol, camphor, methyl salicylate, polysorbate 80 (e.g., TWEEN^{™} 80, Sigma-Aldrich, St. Louis, MO), SDS, benzalkonium chloride, polyoxyl 40 hydrogenated castor oil (e.g., CREMOPHOR^{™} RH40, KOLLIPHOR^{™} RH40, BASF, Germany), didecyldimethylammonium bromide (DDAB), didecyltrimethylammonium bromide (DTAB), fatty acids esters (e.g., isopropyl myristate, isopropyl palmitate), fatty acids (e.g., oleic acid, palmitic acid, linoleic acid, and salts thereof), fatty alcohols (e.g., oleyl alcohol, myristyl alcohol, and stearyl alcohol), medium-chain triglycerides, and any combination thereof. In some aspects, the penetration enhancer can be dimethyl isosorbide. In other aspects, the penetration enhancer can be a mixture comprising dimethyl isosorbide and diethylene glycol monoethyl ether. In other aspects, the penetration enhancer can be dimethyl sulfoxide.

In some aspects, the penetration enhancer can be present in an amount ranging from about 1 wt% to about 30 wt% with respect to the total weight of the pharmaceutical formulation. In other aspects, the penetration enhancer can be present from about 2 wt% to about 20 wt% with respect to the total weight of the pharmaceutical formulation; or from about 3 wt% to about 15 wt% with respect to the total weight of the pharmaceutical formulation or from about 5 wt% to about 10 wt% with respect to the total weight of the pharmaceutical formulation. According to some aspects, the penetration enhancer can be present in the pharmaceutical formulation described herein in an amount of about 1% wt%, about 2 wt%, about 3 wt%, about 4 wt%, about 5 wt%, about 6 wt%, about 7 wt%, about 8 wt%, about 9 wt%, about 10 wt%, about 11 wt%, about 12 wt%, about 13 wt%, about 14 wt%, about 15 wt%, about 16 wt%, about 17 wt%, about 18 wt%, about 19 wt%, about 20 wt%, about 21 wt%, about 22 wt%, about 23 wt%, about 24 wt%, about 25 wt%, about 26 wt%, about 27 wt%, about 28 wt%, about 29 wt%, or about 30 wt% with respect to the total weight of the pharmaceutical formulation.

In some aspects, the pharmaceutical formulation can include one or more moisturizers. Without wishing to be bound by a particular theory, a moisturizer is believed to impart moisture and/or softness into the skin, particularly when a component known to dry the skin, such as an alcohol, are also present in the formulation. In some aspects, the moisturizer can be a fatty alcohol, a fatty acid (e.g., myristic acid, palmitic acid, palmitoleic acid, stearic acid, oleic acid, linoleic acid, linolenic acid, or any combination thereof), a fatty acid ester, an oil, a polyethylene glycol, glycerol, an alpha hydroxy acid, or any combination thereof. In some aspects, the moisturizer can be cetyl alcohol, cetearyl alcohol, stearyl alcohol, stearic acid, glyceryl monostearate, isopropyl myristate, isopropyl palmitate, cocoa butter, lanolin, liquid paraffin (e.g., mineral oil), shea butter, a silicone oil, castor oil, a polyethylene glycol, glycerol, lactic acid, glycolic acid, malic acid, citric acid, tartaric acid, or any combination thereof. In some aspects, the moisturizer can be cetyl alcohol, stearyl alcohol, or a combination of cetyl alcohol and stearyl alcohol. In some aspects, the moisturizer can be cetyl alcohol, liquid paraffin (e.g., mineral oil), or a combination of cetyl alcohol and liquid paraffin.

In some aspects, the pharmaceutical formulation can include the moisturizer in an amount of up to about 15 wt% of the total formulation. In some aspects, the moisturizer can be present in an amount ranging from 1 wt% to 15 wt% (e.g., from about 1 wt% to about 14 wt%, from about 1 wt% to about 13 wt%, from about 1 wt% to about 12 wt%, from about 1 wt% to about 11 wt%, from about 1 wt% to about 10 wt%, from about 1 wt% to about 9 wt%, from about 1 wt% to about 8 wt%, from about 1 wt% to about 7 wt%, from about 1 wt% to about 6 wt%, from about 1 wt% to about 5 wt%, from about 1 wt% to about 4 wt%, from about 1 wt% to about 3 wt%, from about 1 wt% to about 2 wt%, from about 5 wt% to about 15 wt%, from about 5 wt% to about 14 wt%, from about 5 wt% to about 13 wt%, from about 12 wt% to about 10 wt%, from about 10 wt% to about 15 wt%, from about 10 wt% to about 14 wt%, or from about 10 wt% to about 13 wt%,). In some aspects, the pharmaceutical formulation can include the moisturizer in an amount of about 1 wt%, about 2 wt%, about 3 wt%, about 4 wt%, about 5 wt%, about 6 wt%, about 7 wt%, about 8 wt%, about 9 wt%, about 10 wt%, about 11 wt%, about 12 wt%, about 13 wt%, about 14 wt%, or about 15 wt%. In still further aspects, the moisturizer can be present in the pharmaceutical formulation at about 13 wt%.

In some aspects, the pharmaceutical formulation can include one or more preservatives (e.g., an antibacterial, an antifungal). Suitable preservatives include, e.g., a paraben (e.g., methyl paraben, ethyl paraben, propyl paraben, butyl paraben), a benzoate (e.g., sodium benzoate), a sorbate (e.g., potassium sorbate), a quaternary ammonium compound (e.g., benzalkonium chloride, benzethonium chloride), an alcohol (e.g., chlorobutanol, benzyl alcohol, ethanol, phenol, m-cresol, chloro cresol), sodium metabisulfite, imidazolidinyl urea, glycerol, propylene glycol, or any combination thereof.

In some aspects, the pharmaceutical formulation can include the preservative in an amount of up to about 5 wt% of the total formulation (e.g., up to about 4 wt%, up to about 3 wt%, up to about 2 wt%). In some aspects, the preservative can be present in an amount ranging from 0.05 wt% to 5 wt% (e.g., from about 0.05 wt% to about 4 wt%, from about 0.05 wt% to about 3 wt%, from about 0.05 wt% to about 2 wt%). In some aspects, the pharmaceutical formulation can include the preservative in an amount of about 0.1 wt%, about 0.2 wt%, about 0.3 wt%, about 0.4 wt%, about 0.5 wt%, about 0.6 wt%, about 0.7 wt%, about 0.8 wt%, about 0.9 wt%, about 1 wt%, about 1.5 wt%, about 2 wt%, about 2.5 wt%, about 3 wt%, about 3.5 wt%, about 4 wt%, about 4.5 wt%, or about 5 wt%.

In some aspects, the pharmaceutical formulation can include one or more chelating agents. Suitable chelating agents include, e.g., citric acid, phytic acid, sodium phytate, clioquinol, ethylenediaminetetraacetic acid (EDTA), etidronic acid, galactaric acid, tetrahydroxypropyl ethylenediamine, sodium metasilicate, any salt thereof (e.g., an edetate salt, such as disodium EDTA or tetrasodium EDTA), or any combination thereof.

In some aspects, the pharmaceutical formulation can include the chelating agent in an amount of up to about 5 wt% of the total formulation (e.g., up to about 4 wt%, up to about 3 wt%, up to about 2 wt%). In some aspects, the chelating agent can be present in an amount ranging from 0.05 wt% to 5 wt% (e.g., from about 0.05 wt% to about 4 wt%, from about 0.05 wt% to about 3 wt%, from about 0.05 wt% to about 2 wt%). In some aspects, the pharmaceutical formulation can include the chelating agent in an amount of about 0.1 wt%, about 0.2 wt%, about 0.3 wt%, about 0.4 wt%, about 0.5 wt%, about 0.6 wt%, about 0.7 wt%, about 0.8 wt%, about 0.9 wt%, about 1 wt%, about 1.5 wt%, about 2 wt%, about 2.5 wt%, about 3 wt%, about 3.5 wt%, about 4 wt%, about 4.5 wt%, or about 5 wt%.

In some aspects, the pharmaceutical formulation can comprise cortexolone 17α-propionate, a solvent comprising water and a diol, at least one emulsifier, at least one moisturizer, optionally an antioxidant, and optionally a chelating agent. In some aspects, the pharmaceutical formulation can be an emulsified cream.

In some aspects, the pharmaceutical formulation can comprise cortexolone 17α-propionate, propylene glycol, cetyl alcohol, tocopherol, citric acid, edetate disodium, mono- and di-glycerides, mineral oil, polysorbate 80, and water. In some aspects of this formulation, the pharmaceutical formulation can be a cream, such as an emulsified cream.

In some aspects, the pharmaceutical formulation (e.g., an emulsified cream) can comprise, consist essentially of, or consist of:
about 0.1 to about 2 wt% cortexolone 17α-propionate,
about 20 to about 30 wt% propylene glycol,
about 0.5 to about 5 wt% cetyl alcohol,
about 10 to about 20 wt% glyceryl monostearate,
about 5 to about 15 wt% liquid paraffin,
about 0.05 to about 5 wt% polysorbate 80,
about 0 to about 0.1 wt% tocopherol, and
water.

In some aspects, the pharmaceutical formulation (e.g., an emulsified cream) can comprise, consist essentially of, or consist of:
about 1 wt% cortexolone 17α-propionate,
about 25 wt% propylene glycol,
about 2.5 wt% cetyl alcohol,
about 15 wt% glyceryl monostearate,
about 10 wt% liquid paraffin,
about 1 wt% polysorbate 80,
up to about 0.05 wt% tocopherol, and
the remainder water.

In any of the aspects described herein, cortexolone 17α-propionate can be administered as WINLEVI^{™} (Cosmo Pharmaceuticals NV, Ireland). The prescribing information for WINLEVI^{™} is incorporated herein by reference in its entirety.

### Modes of Administration

The pharmaceutical formulations described herein can be administered according to varying schedules. In one aspect, the mode of administration of the pharmaceutical formulation can be continuous. For example, the pharmaceutical formulations can be applied topically once a day, twice daily, three times a day, four times a day, or more, as specified by a physician. In some aspects, a formulation described herein can be applied topically once a day or twice daily. According to a particular aspect, the pharmaceutical formulation described herein can be applied topically once a day. According to another aspect, the pharmaceutical formulation described herein can be applied topically twice daily.

In some aspects, a dosing regimen can be tapered. That is, the pharmaceutical formulation can be applied once a day for a first period of time, twice daily thereafter for a second period of time, three times a day thereafter for a third period of time, and so on. In a particular aspect, the pharmaceutical formulation can be applied once a day on the first day, and twice daily thereafter, with the appropriate duration of treatment determined by, for example, a subject's physician. In an alternative aspect, tapered administration can comprise administering the pharmaceutical formulation in a reducing schedule starting from multiple administrations per day for an appropriate period of time and reducing the number of administrations over an appropriate period of time until a maintenance scheduled can be achieved. It is within the skill of a physician of ordinary skill in the art armed with the present disclosure to determine the appropriate starting point, reducing taper, and maintenance schedule.

In some aspects, the pharmaceutical formulation can be applied over the course of a period of days, weeks, or months. For example, the pharmaceutical formulation can be applied one, two, three, four, or five times a day for up to: 1, 2, 3, 4, 5, 6, or 7 days (i.e., about 1 week); about 2 weeks, about 3 weeks, or about 4 weeks; about 2 months, about 3 months, about 4 months, about 5 months, about 6 months, about 7 months, about 8 months, about 9 months, about 10 months, about 11 months, about 12 months (i.e., about a year), about 13 months, about 14 months, about 15 months, about 16 months, about 17 months, about 18 months, about 19 months, about 20 months, about 21 months, about 22 months, about 23 months, or about 24 months (i.e., about 2 years). According to a particular aspect, the pharmaceutical formulation can be applied once on the first day and twice daily thereafter for about 4 weeks. In another aspects, the pharmaceutical formulation can be topically applied once a day for 1 month, once a day for 2 months, once a day for 3 months, once a day for 4 months, once a day 5 months, once a day for 6 months, once a day for 8 months, once a day for 12 months, once a day for 14 months, once a day for 16 months, once a day for 18 months, once a day for 20 months, once a day for 22 months, or once a day for 24 months. In some aspects, the pharmaceutical formulation can be topically applied to the skin once daily for 6 months. In some aspects, the pharmaceutical formulation can be topically applied to the skin once daily for 12 months.

In other aspects, the pharmaceutical formulation can be topically applied twice daily for: about 1 month; for about 2 months; for about 3 months; for about 4 months; for about 5 months; for about 6 months, for about 8 months, for about 12 months, for about 14 months, for about 16 months, for about 18 months, for about 20 months, for about 22 months, or for about 24 months. In some aspects, the pharmaceutical formulation can be topically applied to the skin twice daily for 6 months. In some aspects, the pharmaceutical formulation can be topically applied to the skin twice daily for 12 months.

In other aspects, the pharmaceutical formulation can be applied more than twice daily (i.e., 3 times a day (TID), 4 times a day (QID), etc.) for: about 1 month; for about 2 months; for about 3 months; for about 4 months; for about 5 months; for about 6 months, for about 8 months, for about 12 months, for about 14 months, for about 16 months, for about 18 months, for about 20 months, for about 22 months, or for about 24 months. In some aspects, the pharmaceutical formulation can be topically applied to the skin more than twice daily (i.e., TID, QID, etc.) for 6 months. In some aspects, the pharmaceutical formulation can be topically applied to the skin more than twice daily (i.e., TID, QID, etc.) for 12 months.

In another aspect, the mode of administration of the pharmaceutical formulations can be cyclic. For example, the pharmaceutical formulation can first be applied in a continuous way as described above for a desired period of time, the application can then be discontinued for a period of time, such as a few days, and then the application of the pharmaceutical formulations can be started again, as described above. The treatment period can include one or more cycles which can be the same or different. In some aspects, the treatment period can be as follows: a) the pharmaceutical formulation can be topically applied for a period of time, such as 4 months, by continuous administration, b) the application can be discontinued for a few days (e.g., 2 to 5 days), and 3) the topical application can be continued for an additional period of time, such as 6 months.

The "effective amount" is an amount that provides a desired outcome (e.g., to treat, ameliorate, and/or prevent acneiform eruptions). In some aspects, the pharmaceutical formulation can comprise cortexolone-17α-propionate at a concentration of from about 0.1 wt% to about 5 wt%. For example, the pharmaceutical formulation can comprise cortexolone-17α-propionate at a concentration of about 0.1 wt%, about 0.2 wt%, about 0.3 wt%, about 0.4 wt%, about 0.5 wt%, about 0.6 wt%, about 0.7 wt%, about 0.8 wt%, about 0.9 wt%, about 1 wt%, about 1.1 wt%, about 1.2 wt%, about 1.3 wt%, about 1.4 wt%, about 1.5 wt%, about 1.6 wt%, about 1.7 wt%, about 1.8 wt%, about 1.9 wt%, about 2 wt%, about 2.1 wt%, about 2.2 wt%, about 2.3 wt%, about 2.4 wt%, about 2.5 wt%, about 2.6 wt%, about 2.7 wt%, about 2.8 wt%, about 2.9 wt%, about 3 wt%, about 3.1 wt%, about 3.2 wt%, about 3.3 wt%, about 3.4 wt%, about 3.5 wt%, about 3.6 wt%, about 3.7 wt%, about 3.8 wt%, about 3.9 wt%, about 4 wt%, about 4.1 wt%, about 4.2 wt%, about 4.3 wt%, about 4.4 wt%, about 4.5 wt%, about 4.6 wt%, about 4.7 wt%, about 4.8 wt%, about 4.9 wt%, or about 5 wt%. In some aspects, the pharmaceutical formulation can comprise cortexolone-17α-propionate at a concentration of about 1 wt%. In some aspects, the pharmaceutical formulation can comprise cortexolone-17α-propionate at a concentration of about 1.5 wt%. In some aspects, the pharmaceutical formulation can comprise cortexolone-17α-propionate at a concentration of about 2 wt%. In some aspects, the pharmaceutical formulation can comprise cortexolone-17α-propionate at a concentration of about 2.5 wt%. In some aspects, the pharmaceutical formulation can comprise cortexolone-17α-propionate at a concentration of about 3 wt%. In some aspects, the pharmaceutical formulation can comprise cortexolone-17α-propionate at a concentration of about 3.5 wt%. In some aspects, the pharmaceutical formulation can comprise cortexolone-17α-propionate at a concentration of about 4 wt%. In some aspects, the pharmaceutical formulation can comprise cortexolone-17α-propionate at a concentration of about 4.5 wt%. In some aspects, the pharmaceutical formulation can comprise cortexolone-17α-propionate at a concentration of about 5 wt%.

In some aspects, the amount of cortexolone-17α-propionate in a single application can range from about 2 mg to about 150 mg. In other aspects, the amount of cortexolone-17α-propionate in a single application can range from about 2 mg to about 145 mg. In other aspects, the amount of cortexolone-17α-propionate in a single application can range from about 2 mg to about 140 mg. In other aspects, the amount of cortexolone-17α-propionate in a single application can range from about 2 mg to about 135 mg. In other aspects, the amount of cortexolone-17α-propionate in a single application can range from about 2 mg to about 130 mg. In other aspects, the amount of cortexolone-17α-propionate in a single application can range from about 2 mg to about 125 mg. In other aspects, the amount of cortexolone-17α-propionate in a single application can range from about 2 mg to about 120 mg. In other aspects, the amount of cortexolone-17α-propionate in a single application can range from about 2 mg to about 115 mg. In other aspects, the amount of cortexolone-17α-propionate in a single application can range from about 2 mg to about 110 mg. In other aspects, the amount of cortexolone-17α-propionate in a single application can range from about 2 mg to about 105 mg. In other aspects, the amount of cortexolone-17α-propionate in a single application can range from about 2 mg to about 100 mg. In other aspects, the amount of cortexolone-17α-propionate in a single application can range from about 2 mg to about 95 mg. In other aspects, the amount of cortexolone-17α-propionate in a single application can range from about 2 mg to about 90 mg. In other aspects, the amount of cortexolone-17α-propionate in a single application can range from about 2 mg to about 85 mg. In other aspects, the amount of cortexolone-17α-propionate in a single application can range from about 2 mg to about 80 mg. In other aspects, the amount of cortexolone-17α-propionate in a single application can range from about 2 mg to about 75 mg. In other aspects, the amount of cortexolone-17α-propionate in a single application can range from about 2 mg to about 70 mg. In other aspects, the amount of cortexolone-17α-propionate in a single application can range from about 2 mg to about 65 mg. In other aspects, the amount of cortexolone-17α-propionate in a single application can range from about 2 mg to about 60 mg. In other aspects, the amount of cortexolone-17α-propionate in a single application can range from about 2 mg to about 55 mg. In other aspects, the amount of cortexolone-17α-propionate in a single application can range from about 2 mg to about 50 mg. In other aspects, the amount of cortexolone-17α-propionate in a single application can range from about 2 mg to about 45 mg. In other aspects, the amount of cortexolone-17α-propionate in a single application can range from about 2 mg to about 40 mg. In other aspects, the amount of cortexolone-17α-propionate in a single application can range from about 2 mg to about 35 mg. In other aspects, the amount of cortexolone-17α-propionate in a single application can range from about 2 mg to about 30 mg. In other aspects, the amount of cortexolone-17α-propionate in a single application can range from about 2 mg to about 25 mg. In some aspects, the amount of cortexolone-17α-propionate in a single application can range from about 2 mg to about 20 mg. In other aspects, the amount of cortexolone-17α-propionate in a single application can range from about 2 mg to about 17 mg. In other aspects, the amount of cortexolone-17α-propionate in a single application can range from about 2 mg to about 15 mg. In some aspects, the amount of cortexolone-17α-propionate in a single application can range from about 2 mg to about 10 mg. In some aspects, the amount of cortexolone-17α-propionate in a single application can be about 2 mg, about 2.5 mg, about 3 mg, about 3.5 mg, about 4 mg, about 4.5 mg, about 5 mg, about 5.5 mg, about 6 mg, about 6.5 mg, about 7 mg, about 7.5 mg, about 8 mg, about 8.5 mg, about 9 mg, about 10 mg, about 11 mg, about 12 mg, about 13 mg, about 14 mg, about 15 mg, about 16 mg, about 17 mg, about 18 mg, about 19 mg, about 20 mg, about 21 mg, about 22 mg, about 23 mg, about 24 mg, about 25 mg, about 26 mg, about 27 mg, about 28 mg, about 29 mg, about 30 mg, about 31 mg, about 32 mg, about 33 mg, about 34 mg, about 35 mg, about 36 mg, about 37 mg, about 38 mg, about 39 mg, about 40 mg, about 41 mg, about 42 mg, about 43 mg, about 44 mg, about 45 mg, about 46 mg, about 47 mg, about 48 mg, about 49 mg, about 50 mg, about 51 mg, about 52 mg, about 53 mg, about 54 mg, about 55 mg, about 56 mg, about 57 mg, about 58 mg, about 59 mg, about 60 mg, about 61 mg, about 62 mg, about 63 mg, about 64 mg, about 65 mg, about 66 mg, about 67 mg, about 68 mg, about 69 mg, about 70 mg, about 71 mg, about 72 mg, about 73 mg, about 74 mg, about 75 mg, about 76 mg , about 77 mg, about 78 mg, about 79 mg, about 80 mg, about 81 mg, about 82 mg, about 83 mg, about 84 mg, about 85 mg, about 86 mg, about 87 mg, about 88 mg, about 89 mg, about 90 mg, about 91 mg, about 92 mg, about 93 mg, about 94 mg, about 95 mg, about 96 mg, about 97 mg, about 98 mg, about 99 mg, or about 100 mg. In another aspect, the amount of cortexolone-17α-propionate in a single application can be about 10 mg. In another aspect, the amount of cortexolone-17α-propionate in a single application can be about 20 mg. In yet another aspect, the amount of cortexolone-17α-propionate in a single application can be about 30 mg. In yet another aspect, the amount of cortexolone-17α-propionate in a single application can be about 40 mg. In yet another aspect, the amount of cortexolone-17α-propionate in a single application can be about 50 mg.

In some aspects, the pharmaceutical formulation can be self-administered by the subject once a day or twice daily. In some aspects, when the pharmaceutical formulation is a cream or in liquid form having a cortexolone-17α-propionate concentration of about 1 wt%, the pharmaceutical formulation can be self-administered once a day or twice daily at a dose ranging from about 0.2 to about 2.0 ml, from about 0.5 to about 1.5 ml, and in further aspects at about 1 mL or about 1.5 mL.

The pharmaceutical formulation described herein can be applied to any body surface in need of treatment, such as a skin region susceptible to acneiform eruptions. Suitable skin regions include e.g., face, arm, armpit, forearm, leg, chest, trunk, shoulder, abdomen, back, neck, or any combination of the foregoing. In some aspects, the formulation is not delivered to the face. In other aspects, the formulation can be applied to the face.

In some aspects, the method of treating acneiform eruptions can comprise administering a pharmaceutical formulation, as described herein, comprising a combination of an effective amount of cortexolone-17α-propionate and one or more secondary anti-acneiform agents. In some aspects, one, two, three, or four anti-acneiform agents can be administered in combination with cortexolone-17α-propionate as a formulation or administered sequentially.

The secondary anti-acneiform agent can be selected from the group consisting of an antiseptic, an antibiotic, a retinoid, an antioxidant, an organic acid, a corticosteroid, an antiparasitic agent, sulfur, urea, and combinations thereof.

### Storage Stability

Storage stability is an important metric for pharmaceutical products. In general, greater stability means that a given formulation is both easier to transport and store, increasing the likelihood that it will be stocked by pharmacies and that subjects will not have to be concerned with special storage instructions. In some aspects, the pharmaceutical formulation described herein can have a desirable stability profile allowing for storage of the final formulation for at least about 3 months, at least about 6 months, at least about 9 months, at least about 12 months, at least about 15 months, at least about 18 months, at least about 21 months, or at least about two years - each at room (about 20 °C) or refrigerated temperatures (e.g., about 4 °C).

For example, one of the main degradation pathways of cortexolone-17α-propionate is transesterification to cortexolone-21-propionate (17a-hydroxy-21-propionyloxy-pregna-4-ene-3,20-dione):

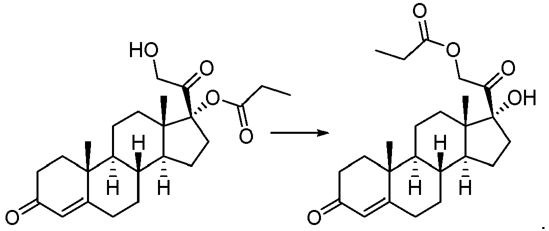

In some aspects, maintaining the pharmaceutical formulations disclosed herein at an acidic pH, including a pH of less than about 7.0 (e.g., less than about 6.5, less than about 6, less than about 5.5, less than about 5, less than about 4.5, between about 4 and about 7, between about 4 and about 6.5, between about 4 and about 6, between about 4 and 5.5, between about 4 and 5, between about 4 and about 4.5, or at about 6.5, about 6, about 5.5, about 5, about 4.5, or about 4), can slow this degradation process. In some aspects, the pH can be about 4. The appropriate pH can be obtained via addition of a suitable amount of a pH modifier, such as the acids described herein.

In some aspects, the pharmaceutical formulations described herein can, at room (about 20 °C) or refrigerated (e.g., about 4 °C) temperatures, have less than about 5 wt% cortexolone-21-propionate or other degradation products after storage for a period of about 24 months.

The present disclosure is further illustrated by the following embodiments.
(1) Cortexolone-17α-propionate for use in the treatment of a cutaneous acneiform eruption in a subject in need thereof, characterized in that it is administered topically to an affected area of the subject.
(2) Cortexolone-17α-propionate for use according to (1), wherein the acneiform eruption comprises a papule, a pustule, a nodule, crust, a cyst, or any combination thereof on the affected area of the subject.
(3) Cortexolone-17a-propionate for use according to (1), wherein the acneiform eruption is a targeted therapeutic drug-induced lesion.
(4) Cortexolone-17α-propionate for use according to (1), wherein the subject is receiving treatment for cancer.
(5) Cortexolone-17α-propionate for use according to (3), wherein the treatment comprises a targeted biologic agent selected from the group consisting of an Epidermal Growth Factor Receptor (EGFR) inhibitor, a Mitogen-Activated Protein Kinase (MEK) inhibitor, a Human Epidermal Growth Factor Receptor (HER-2) inhibitor, a Vascular Endothelial Growth Factor Receptor (VEGFR) inhibitor, a RAS/RAF/MEK/ERK (MAPK) inhibitor, a multikinase inhibitor, a mammalian Target of Rapamycin (mTOR) inhibitor, a Programmed Death (PD-1)/Programmed Death Ligand (PDL-1 and PDL-2) inhibitor, a Cytotoxic T Lymphocyte-Associated Antigen (CTLA-4) inhibitor, an Anaplastic Lymphoma Kinase (ALK) inhibitor, a Raf kinase (RAF) inhibitor, a Phosphoinositide 3-kinase (PI3K) inhibitor, and an AKT kinase inhibitor, or any combination thereof, preferably said targeted biologic agent is gefitinib, erlotinib, afatinib, osimertinib, dacomitinib, lapatinib, neratinib, avitinib, olmutinib, pelitinib, pyrotinib, brigatinib, vandetanib, icotinib, mobocertinib, almonertinib, lazertinib, alflutinib, rociletinib, nazartinib, naquotinib, cetuximab, panitumumab, necitumumab, amivantamab, trametinib, cobimetinib, pimasertib, mirdametinib, refametinib, binimetinib, selumetinib, trastuzumab, pertuzumab, bevacizumab, aflibercept, vemurafenib, dabrafenib, encorafenib, pictilisib, copanlisib, buparlisib, taselisib, ipatasertib, uprosertib, sorafenib, sunitinib, everolimus, sirolimus, nivolumab, pembrolizumab, ipilimumab, tremelimumab, or any combination thereof.
(6) Cortexolone-17α-propionate for use according to any one of (1)-(5), wherein the cortexolone-17α-propionate is administered in a pharmaceutical formulation comprising a pharmaceutically acceptable carrier, preferably said pharmaceutical formulation is a solution, a suspension, an emulsion, a microemulsion, a cream, a biogel, a paste, a gel, a foam, an ointment, a wash-off agent, a patch, a hydrogel patch, or a pledget.
(7) Cortexolone-17α-propionate for use according to (6), wherein the pharmaceutical formulation further comprises at least one additive selected from the group consisting of an acid, a buffer, an antioxidant, an emulsifier, a penetration enhancer, a moisturizer, a preservative, a chelating agent, and any combination thereof.
(8) Cortexolone-17α-propionate for use according to (7), wherein the antioxidant is selected from the group consisting of butylated hydroxytoluene (BHT), butylated hydroxyanisole (BHA), ascorbyl palmitate, ascorbic acid, tocopherol, propyl gallate, 2,4,5-trihydroxybutyrophenone, 4-hydroxymethyl-2,6-di-tert-butylphenol, erythorbic acid, gum guaiac, thiodipropionic acid, dilauryl thiodipropionate, tert-butylhydroquinone, pharmaceutically acceptable salts thereof, esters thereof, and any combination thereof.
(9) Cortexolone-17α-propionate for use according to (7) or (8), wherein the emulsifier is selected from the group consisting of PEG-15 hydroxystearate (polyoxyl-15-hydroxystearate), PEG-30 stearate, PEG-40 laurate, PEG-40 oleate, polysorbate 20, polysorbate 60, polysorbate 80, PEG-20 cetostearyl ether, polyoxyl 25 cetostearyl, glyceryl monostearate, cetomacrogol 1000, sorbitan monolaurate, sorbitan monopalmitate, sorbitan monooleate a propylene glycol ester of a fatty acid, a polyglycerol ester of a fatty acid, polyoxyl 5 castor oil, polyoxyl 15 castor oil, polyoxyl 35 castor oil, polyoxyl 40 hydrogenated castor oil, caprylocapryl polyoxyl-8 glycerides, caprylocaproyl polyoxylglycerides, lauroyl polyoxylglycerides, oleoyl polyoxylglycerides, and any combination thereof.
(10) Cortexolone-17α-propionate for use according to (9), wherein the emulsifier is polysorbate 80, glyceryl monostearate, or a combination of polysorbate 80 and glyceryl monostearate.
(11) Cortexolone-17α-propionate for use according to any one of (7)-(10), wherein the penetration enhancer is selected from the group consisting of a diol, a polyol, a fatty acid, a fatty alcohol, a fatty acid ester, a surfactant, a pyrrolidone, and any combination thereof.
(12) Cortexolone-17a-propionate for use according to any one of (7)-(11), wherein the moisturizer is cetyl alcohol, cetearyl alcohol, stearyl alcohol, stearic acid, glyceryl monostearate, isopropyl myristate, isopropyl palmitate, cocoa butter, lanolin, liquid paraffin, shea butter, a silicone oil, castor oil, a polyethylene glycol, glycerol, lactic acid, glycolic acid, malic acid, citric acid, tartaric acid, or any combination thereof.
(13) Cortexolone-17a-propionate for use according to any one of (7)-(12), wherein the buffer is a phosphate buffer, citrate buffer, lactate buffer, or any combination thereof.
(14) Cortexolone-17α-propionate for use according to any one of (6) or (7), wherein the pharmaceutical formulation comprises propylene glycol, cetyl alcohol, tocopherol, citric acid, edetate disodium, mono- and di-glycerides, mineral oil, polysorbate 80, and water.
(15) Cortexolone-17α-propionate for use according to (14), wherein the pharmaceutical formulation comprises:
   about 1 wt% cortexolone 17α-propionate,
   about 25 wt% propylene glycol,
   about 2.5 wt% cetyl alcohol,
   about 15 wt% glyceryl monostearate,
   about 10 wt% liquid paraffin,
   about 1 wt% polysorbate 80, and
   up to about 0.05 wt% tocopherol.
(16) Cortexolone-17α-propionate for use according to any one of (1)-(15), wherein the cortexolone-17α-propionate is administered in an amount of about 0.1 to about 5 wt%, preferably the cortexolone 17α-propionate is administered in an amount of about 1 wt%.
(17) Cortexolone-17a-propionate for use according to any one of (1)-(16), wherein topically applying the cortexolone 17α-propionate comprises administering a formulation comprising cortexolone 17α-propionate dissolved therein and cortexolone 17α-propionate present in at least one of polymorphic Forms I, II, III, and IV, or any combination thereof.

### EXAMPLES

The pharmaceutical formulations described herein are now further detailed with reference to the following examples. These examples are provided for the purpose of illustration only and the aspects described herein should in no way be construed as being limited to these examples. Rather, the aspects should be construed to encompass any and all variations which become evident as a result of the teaching provided herein.

### Example 1: Pharmaceutical Formulation of Cortexolone-17α-propionate

One hundred g of cortexolone 17α-propionate of crystalline Form III was dissolved in 2500 g of propylene glycol under stirring at room temperature. An emulsion with 250 g of cetyl alcohol, 1500 g of glyceryl monostearate, 1000 g of liquid paraffin (e.g., mineral oil), 5 g of mixed tocopherols (e.g., vitamin E), 100 g of polysorbate 80, and 4650 g of purified water was separately prepared by using a turboemulsifier, raising the temperature up to about 70 °C. After cooling the emulsion to about 30 °C, the cortexolone 17α-propionate solution was added, under stirring and under negative pressure, in propylene glycol. The emulsioned cream was maintained under stifling heat until homogeneity was obtained, making sure the temperature remained low by means of circulation of a coolant.

The cream contained a dispersed crystalline fraction, made up of an active ingredient in solvate crystalline Form IV, formed due to the precipitation of the active ingredient itself from the glycolic solution which contained it when the latter was added to the predominantly aqueous formulation.

### Example 2: Method of Treating Acneiform Eruptions

A patient being treated with a targeted therapeutic drug, such as an EGFR inhibitor, presents to a physician with acneiform eruptions including pustules and crusts with pruritus and tenderness, which is scored as Grade 3 acneiform eruptions. A physician of skill in the art instructs the patient to self-administer the pharmaceutical formulation of Example 1 topically to the affected area twice a day for 4 weeks. After about two weeks of treatment with the pharmaceutical formulation, it is expected that the patient will present with improved acneiform eruptions scored as Grade 2. After about four weeks of treatment with the pharmaceutical formulation, it is expected that the patient will present with further improved acneiform eruptions scored as Grade 1. In the following weeks, it is expected that the patient's acneiform eruptions will be cleared.

The example suggests that cortexolone 17α-propionate applied to acneiform eruptions induced by EGFR inhibitors can treat affected areas of the subject where the pharmaceutical formulation is topically administered.

### Example 3: Method of Preventing Acneiform Eruptions

A patient is diagnosed with cancer and prescribed an EGFR inhibitor, such as panitumumab or cetuximab. To prevent acneiform eruptions induced by the EGFR inhibitor, the patient is instructed to self-administer the pharmaceutical composition of Example 1 topically to any suitable skin area (e.g., face, chest) twice a day for 4 weeks. At the initiation of cancer treatment, the patient presents with no rash or cutaneous eruptions. After 4 weeks of treatment with the EGFR inhibitor and the pharmaceutical composition of Example 1, it is expected that the patient may report a rash or cutaneous symptoms on an area where the pharmaceutical formulation was not applied, but will have no presentation of acneiform eruptions on the area(s) where the pharmaceutical formulation was applied.

The example suggests that cortexolone 17α-propionate applied at the time of EGFR inhibitor treatment can prevent acneiform eruption in the area where the pharmaceutical formulation is topically administered.

The phraseology or terminology herein is for the purpose of description and not of limitation. As such, the terminology and/or phraseology of the present specification should be interpreted by the skilled artisan in light of the teachings and guidance herein.

The breadth and scope of the present invention should not be limited by any of the above-described exemplary aspects, but should be defined only in accordance with the following claims and their equivalents.

All patents, patent applications, and other references noted or referenced in this application are hereby incorporated by reference in their entirety.

## Claims

1. Cortexolone-17α-propionate for use in the treatment of a cutaneous acneiform eruption in a subject in need thereof **characterized in that** it is administered topically to an affected area of the subject.

2. Cortexolone-17α-propionate for use according to claim 1, wherein the acneiform eruption comprises a papule, a pustule, a nodule, crust, a cyst, or any combination thereof on the affected area of the subject.

3. Cortexolone-17α-propionate for use according to claim 1, wherein the acneiform eruption is a targeted therapeutic drug-induced lesion.

4. Cortexolone-17α-propionate for use according to claim 1, wherein the subject is receiving treatment for cancer.

5. Cortexolone-17α-propionate for use according to claim 3, wherein the treatment comprises a targeted therapeutic drug selected from the group consisting of an Epidermal Growth Factor Receptor (EGFR) inhibitor, a Mitogen-Activated Protein Kinase (MEK) inhibitor, a Human Epidermal Growth Factor Receptor (HER-2) inhibitor, a Vascular Endothelial Growth Factor Receptor (VEGFR) inhibitor, a RAS/RAF/MEK/ERK (MAPK) inhibitor, a multikinase inhibitor, a mammalian Target of Rapamycin (mTOR) inhibitor, a Programmed Death (PD-1)/Programmed Death Ligand (PDL-1 and PDL-2) inhibitor, a Cytotoxic T Lymphocyte-Associated Antigen (CTLA-4) inhibitor, an Anaplastic Lymphoma Kinase (ALK) inhibitor, a Raf kinase (RAF) inhibitor, a Phosphoinositide 3-kinase (PI3K) inhibitor, and an AKT kinase inhibitor, or any combination thereof, preferably said targeted therapeutic drug is gefitinib, erlotinib, afatinib, osimertinib, dacomitinib, lapatinib, neratinib, avitinib, olmutinib, pelitinib, pyrotinib, brigatinig, vandetanib, icotinib, mobocertinib, almonertinib, lazertinib, alflutinib, rociletinib, nazartinib, naquotinib, cetuximab, panitumumab, necitumumab, amivantamab, trametinib, cobimetinib, pimasertib, mirdametinib, refametinib, binimetinib, selumetinib, trastuzumab, pertuzumab, bevacizumab, aflibercept, vemurafenib, dabrafenib, encorafenib, pictilisib, copanlisib, buparlisib, taselisib, ipatasertib, uprosertib, sorafenib, sunitinib, everolimus, sirolimus, nivolumab, pembrolizumab, ipilimumab, tremelimumab, or any combination thereof.

6. Cortexolone-17α-propionate for use according to any one of claims 1-5, wherein the cortexolone-17α-propionate is administered in a pharmaceutical formulation comprising a pharmaceutically acceptable carrier, preferably said pharmaceutical formulation is a solution, a suspension, an emulsion, a microemulsion, a cream, a paste, a biogel, a gel, a foam, an ointment, a wash-off agent, a patch, a hydrogel patch, or a pledget.

7. Cortexolone-17a-propionate for use according to claim 6, wherein the pharmaceutical formulation further comprises at least one additive selected from the group consisting of an acid, a buffer, an antioxidant, an emulsifier, a penetration enhancer, a moisturizer, a preservative, a chelating agent, and any combination thereof.

8. Cortexolone-17α-propionate for use according to claim 7, wherein the antioxidant is selected from the group consisting of butylated hydroxytoluene (BHT), butylated hydroxyanisole (BHA), ascorbyl palmitate, ascorbic acid, tocopherol, propyl gallate, 2,4,5-trihydroxybutyrophenone, 4-hydroxymethyl-2,6-di-tert-butylphenol, erythorbic acid, gum guaiac, thiodipropionic acid, dilauryl thiodipropionate, tert-butylhydroquinone, pharmaceutically acceptable salts thereof, esters thereof, and any combination thereof.

9. Cortexolone-17a-propionate for use according to claim 7 or 8, wherein the emulsifier is selected from the group consisting of PEG-15 hydroxystearate (polyoxyl-15-hydroxystearate), PEG-30 stearate, PEG-40 laurate, PEG-40 oleate, polysorbate 20, polysorbate 60, polysorbate 80, PEG-20 cetostearyl ether, polyoxyl 25 cetostearyl, glyceryl monostearate, cetomacrogol 1000, sorbitan monolaurate, sorbitan monopalmitate, sorbitan monooleate a propylene glycol ester of a fatty acid, a polyglycerol ester of a fatty acid, polyoxyl 5 castor oil, polyoxyl 15 castor oil, polyoxyl 35 castor oil, polyoxyl 40 hydrogenated castor oil, caprylocapryl polyoxyl-8 glycerides, caprylocaproyl polyoxylglycerides, lauroyl polyoxylglycerides, oleoyl polyoxylglycerides, and any combination thereof.

10. Cortexolone-17α-propionate for use according to claim 9, wherein the emulsifier is polysorbate 80, glyceryl monostearate, or a combination of polysorbate 80 and glyceryl monostearate.

11. Cortexolone-17α-propionate for use according to any one of claims 7-10, wherein the penetration enhancer is selected from the group consisting of a diol, a polyol, a fatty acid, a fatty alcohol, a fatty acid ester, a surfactant, a pyrrolidone, and any combination thereof.

12. Cortexolone-17α-propionate for use according to any one of claims 7-11, wherein the moisturizer is cetyl alcohol, cetearyl alcohol, stearyl alcohol, stearic acid, glyceryl monostearate, isopropyl myristate, isopropyl palmitate, cocoa butter, lanolin, liquid paraffin, shea butter, a silicone oil, castor oil, a polyethylene glycol, glycerol, lactic acid, glycolic acid, malic acid, citric acid, tartaric acid, or any combination thereof.

13. Cortexolone-17α-propionate for use according to any one of claims 7-12, wherein the buffer is a phosphate buffer, citrate buffer, lactate buffer, or any combination thereof.

14. Cortexolone-17α-propionate for use according to any one of claims 6 or 7, wherein the pharmaceutical formulation comprises propylene glycol, cetyl alcohol, tocopherol, citric acid, edetate disodium, mono- and di-glycerides, mineral oil, polysorbate 80, and water.

15. Cortexolone-17α-propionate for use according to claim 14, wherein the pharmaceutical formulation comprises:
about 1 wt% cortexolone 17α-propionate,
about 25 wt% propylene glycol,
about 2.5 wt% cetyl alcohol,
about 15 wt% glyceryl monostearate,
about 10 wt% liquid paraffin,
about 1 wt% polysorbate 80, and
up to about 0.05 wt% tocopherol.

16. Cortexolone-17α-propionate for use according to any one of claims 1-15, wherein the cortexolone-17α-propionate is administered in an amount of about 0.1 to about 5 wt%, preferably the cortexolone 17α-propionate is administered in an amount of about 1 wt%.

17. Cortexolone-17α-propionate for use according to any one of claims 1-16, wherein topically applying the cortexolone 17α-propionate comprises administering a formulation comprising cortexolone 17α-propionate dissolved therein and cortexolone 17α-propionate present in at least one of polymorphic Forms I, II, III, and IV, or any combination thereof.
